# EUROPEAN PATENT SPECIFICATION

(11) **EP 2 185 691 B1**
(45) Date of publication and mention of the grant of the patent: **14.03.2018**
(21) Application number: 08837420.2
(22) Date of filing: 31.07.2008
(51) Int. Cl.: C12N 5/071

(54) **PLURIPOTENT STEM CELL DIFFERENTIATION BY USING HUMAN FEEDER CELLS**
DIFFERENZIERUNG PLURIPOTENTER STAMMZELLEN DURCH VERWENDUNG MENSCHLICHER FEEDERZELLEN
DIFFÉRENCIATION DE CELLULES SOUCHES PLURIPOTENTES À L'AIDE DE CELLULES D'ALIMENTATION HUMAINES

(30) Priority: 31.07.2007 US 952937 P
(43) Date of publication of application: 19.05.2010
(60) Divisional application: 12182658.0
(73) Proprietor: LifeScan, Inc., Milpitas, CA 95035 (US)
(72) Inventor: O'NEIL, John, J., Belle Mead, NJ 08502 (US)
(74) Representative: Goodfellow, Hugh Robin
(86) International application number: PCT/US2008/071775
(87) International publication number: WO 2009/048675

(56) References cited:
- WO-A-2006/016999
- WO-A-2007/082963
- WO-A-2007/103282
- INZUNZA J ET AL: "Derivation of human embryonic stem cell lines in serum replacement medium using postnatal human fibroblasts as feeder cells" STEM CELLS, ALPHAMED PRESS, DAYTON, OH, US, vol. 23, no. 4, 1 April 2005 (2005-04-01), pages 544-549, XP002389652 ISSN: 1066-5099
- RICHARDS M ET AL: "Comparative evaluation of various human feeders for prolonged undifferentiated growth of human embryonic stem cells" STEM CELLS, ALPHAMED PRESS, DAYTON, OH, US, vol. 21, no. 5, 1 January 2003 (2003-01-01), pages 546-556, XP002376916 ISSN: 1066-5099
- LEE J B ET AL: "'Establishment and Maintenance of Human Embryonic Stem Cell Lines on Human Feeder Cells Derived from Uterine Endometricum under Serum-Free Condition'" BIOLOGY OF REPRODUCTION, SOCIETY FOR THE STUDY OF REPRODUCTION, CHAMPAIGN, IL, US, vol. 72, 1 January 2005 (2005-01-01), pages 42-49, XP008083585 ISSN: 0006-3363
- D'AMOUR K A ET AL: "Efficient differentiation of human embryonic stem cells to definitive endoderm" NATURE BIOTECHNOLOGY, NATURE PUBLISHING GROUP, NEW YORK, NY, US, vol. 23, no. 12, 28 October 2005 (2005-10-28), pages 1534-1541, XP002385437 ISSN: 1087-0156
- D'AMOUR K A ET AL: "Production of pancreatic hormone-expressing endocrine cells from human embryonic stem cells" NATURE BIOTECHNOLOGY, NATURE PUBLISHING GROUP, NEW YORK, NY, US, vol. 24, no. 11, 1 January 2006 (2006-01-01), pages 1392-1401, XP002423119 ISSN: 1087-0156

## Description

The present invention relates to the field of pluripotent stem cell differentiation. The present invention provides methods for the differentiation of pluripotent stem cells on a human feeder cell layer. In particular, the present invention provides an improved method for the differentiation of pluripotent stem cells into pancreatic endocrine cells using a human feeder cell layer.

### BACKGROUND

Pluripotent stem cells, such as, for example, embryonic stem cells have the ability to differentiate into all adult cell types. As such, embryonic stem cells may be a source of replacement cells and tissue for organs that have been damaged as a result of disease, infection, or congenital abnormalities. The potential for embryonic stem cells to be employed as a replacement cell source is hampered by the difficulty of efficiently differentiating the embryonic stem cells into the cell type of choice.

In one example, Hori et al. (PNAS 99: 16105, 2002) disclose that treatment of mouse embryonic stem cells with inhibitors ofphosphoinositide 3-kinase (LY294002) produced cells that resembled β cells.

In another example, Blyszczuk et al. (PNAS 100:998, 2003) reports the generation of insulin-producing cells from mouse embryonic stem cells constitutively expressing Pax4.

Micallef et al. reports that retinoic acid can regulate the commitment of embryonic stem cells to form Pdx1 positive pancreatic endoderm (Diabetes 54:301, 2005).

Skoudy et al. reports that activin A (a member of the TGFβ superfamily) upregulates the expression of exocrine pancreatic genes (p48 and amylase) and endocrine genes (Pdxl, insulin, and glucagon) in mouse embryonic stem cells (Biochem. J. 379: 749, 2004).

Shiraki et al. studied the effects of growth factors that specifically enhance differentiation of embryonic stem cells into Pdxl positive cells. They observed that TGFβ2 reproducibly yielded a higher proportion of Pdx1 positive cells (Genes Cells. 2005 Jun; 10(6): 503-16.).

Gordon *et al.* demonstrated the induction of brachyury⁺/HNF-3beta⁺ endoderm cells from mouse embryonic stem cells in the absence of serum and in the presence of activin along with an inhibitor of Wnt signaling (US 2006/0003446A1).

Gordon et al. (PNAS 103: 16806, 2006) states "Wnt and TGF-beta/ nodal/ activin signaling simultaneously were required for the generation of the anterior primitive streak".

Thomson et al. isolated embryonic stem cells from human blastocysts (Science 282:114, 1998). Concurrently, Gearhart and coworkers derived human embryonic germ (hEG) cell lines from fetal gonadal tissue (Shamblott et al., Proc. Natl. Acad. Sci. USA 95:13726, 1998). Unlike mouse embryonic stem cells, which can be prevented from differentiating simply by culturing with Leukemia Inhibitory Factor (LIF), human embryonic stem cells must be maintained under very special conditions (U.S. Pat. No. 6,200,806; WO 99/20741; WO 01/51616).

D'Amour et al. describes the production of enriched cultures of human embryonic stem cell-derived definitive endoderm in the presence of a high concentration of activin and low serum (Nature Biotechnology 2005). Transplanting these cells under the kidney capsule of mice resulted in differentiation into more mature cells with characteristics of some endodermal organs. Human embryonic stem cell-derived definitive endoderm cells can be further differentiated into Pdxl positive cells after addition of FGF-10 (US 2005/0266554A1).

D'Amour et al. (Nature Biotechnology - 24, 1392 - 1401 (2006)) states: "We have developed a differentiation process that converts human embryonic stem (hES) cells to endocrine cells capable of synthesizing the pancreatic hormones insulin, glucagon, somatostatin, pancreatic polypeptide and ghrelin. This process mimics *in vivo* pancreatic organogenesis by directing cells through stages resembling definitive endoderm, gut-tube endoderm, pancreatic endoderm and endocrine precursor en route to cells that express endocrine hormones".

In another example, Fisk *et al.* reports a system for producing pancreatic islet cells from human embryonic stem cells (US2006/0040387A1). In this case, the differentiation pathway was divided into three stages. Human embryonic stem cells were first differentiated to endoderm using a combination of sodium butyrate and activin A. The cells were then cultured with TGFβ antagonists such as Noggin in combination with EGF or betacellulin to generate Pdxl positive cells. The terminal differentiation was induced by nicotinamide.

In one example, Benvenistry *et al.* states: "We conclude that over-expression of Pdx1 enhanced expression of pancreatic enriched genes, induction of insulin expression may require additional signals that are only present *in vivo*" (Benvenistry et al, Stem Cells 2006; 24:1923-1930).

In another example, Condie *et al.* disclose: "feeder layers that contain or express ligands or other compounds that inhibit gamma-secretase or Notch signaling to enhance the maintenance of pluripotent cells in a pluripotent state feeder layers that contain or express ligands or other compounds that inhibit gamma-secretase or Notch signaling to enhance the maintenance of pluripotent cells in a pluripotent state" (WO2004090110).

In another example, Mitalipova *et al.* disclose: "Human embryonic stem cells are cultured with human granulosa feeder cells, muscle cells, Fallopian ductal epithelial cells, bone marrow stromal cells, and skin fibroblasts and the embryonic stem cells maintain their pluripotent phenotype" (US20050037488).

in another example, Xu *et al.* disclose: "mesenchymal and fibroblast-like cell lines obtained from embryonic tissue or differentiated from embryonic stem cells. Methods for deriving such cell lines, processing media, and growing stem cells using the feeder cells or conditioned media are described" (US20020072117).

Therefore, there still remains a significant need to develop conditions for establishing pluripotent stem cell lines that can be expanded to address the current clinical needs, while retaining the potential to differentiate into pancreatic endocrine cells, pancreatic hormone expressing cells, or pancreatic hormone secreting cells. We have taken an alternative approach to improve the efficiency of differentiating pluripotent stem cells toward pancreatic endocrine cells.

### SUMMARY

The present invention relates to the field of pluripotent stem cell differentiation. The present invention provides methods for the differentiation of pluripotent stem cells on a human feeder cell layer. In particular, the present invention provides an improved method for the differentiation of pluripotent stem cells into pancreatic endocrine cells using a human feeder cell layer.

In one embodiment, the present invention provides a method for differentiating pluripotent stem cells, according to the claims, comprising the steps of:
a. Culturing the pluripotent stem cells,
b. Plating the pluripotent stem cells onto a human feeder cell layer, and
c. Treating the pluripotent stem cells with at least one factor that promotes the differentiation of the pluripotent stem cells.

### BRIEF DESCRIPTION OF THE DRAWINGS

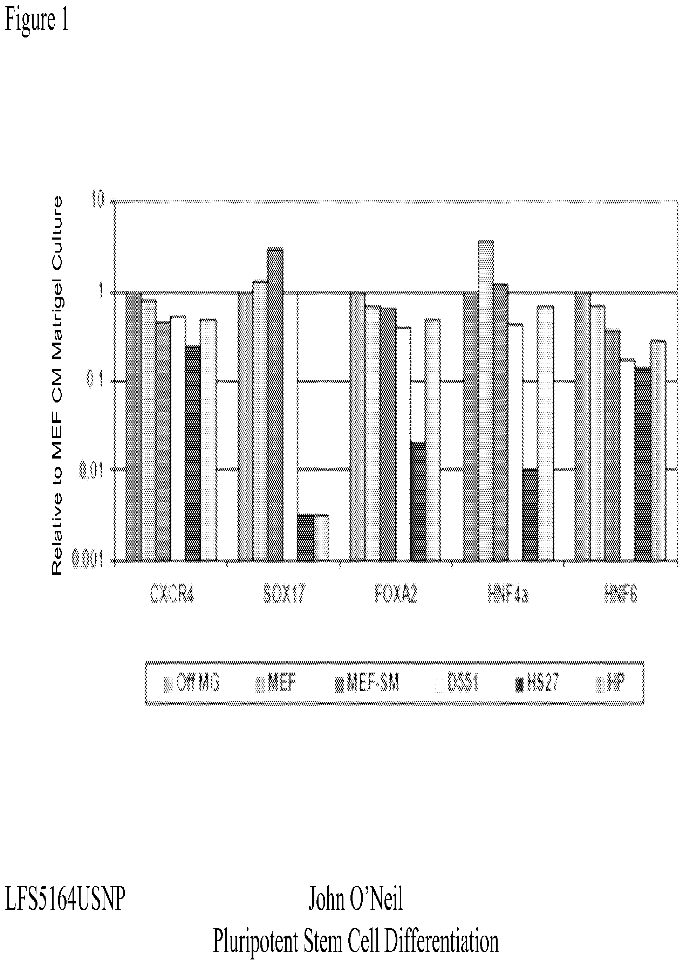

Figure 1 shows the expression of markers associated with differentiation: CXCR4, Sox-17, FoxA2, HNF-4a, HNF6 and AFP in populations of the human embryonic stem cell line H9, at passage 46, cultured on MATRIGEL with MEF conditioned media and compared to cells transferred to mouse embryonic fibroblasts (MEF), commercially available moue embryonic fibroblasts (MEF-SM), human dermal fibroblasts (D551), human foreskin fibroblasts (Hs27), and human pancreatic-derived stromal cells (HP).

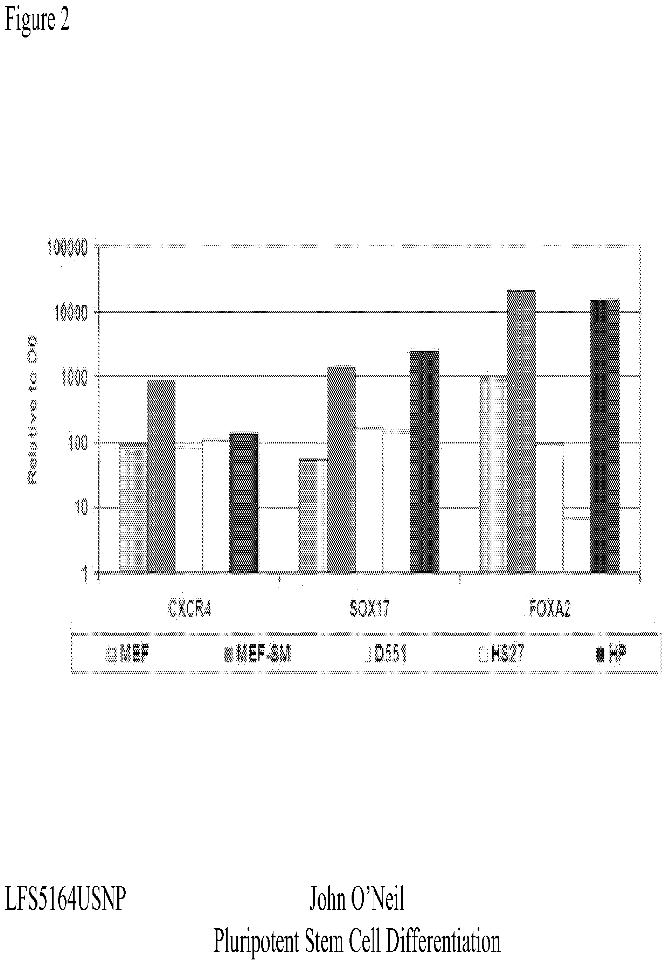

Figure 2 shows the effects of human feeder cell layers on the differentiation of human embryonic stem cells into cells expressing markers characteristic of the definitive endoderm lineage. The figure shows expression of CXCR4, Sox-17, and FoxA2, as determined by real-time PCR in populations of the human embryonic stem cell line H1, at passage 48, differentiated into cells expressing markers characteristic of the definitive endoderm lineage, cultured on mouse embryonic fibroblasts (MEF), commercially available mouse embryonic fibroblasts (MEF-SM), human dermal fibroblasts (D551), human foreskin fibroblasts (Hs27), and human pancreatic-derived stromal cells (HP).

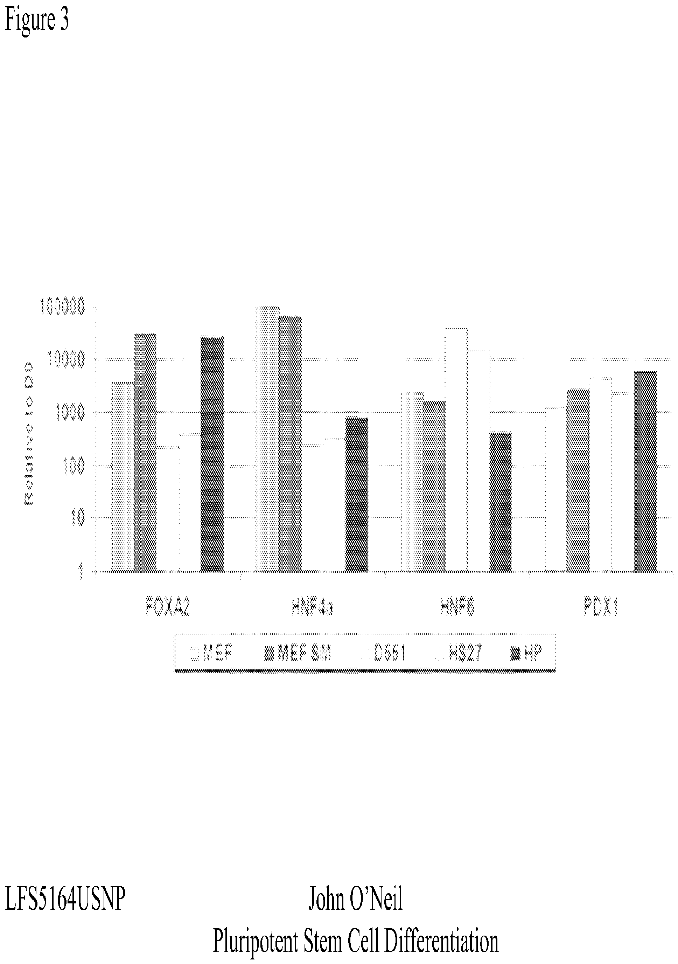

Figure 3 shows the effects of human feeder cell layers on the formation of cells expressing markers characteristic of the pancreatic endoderm lineage. The figure shows expression of FoxA2, HNF-4a, HNF-6 and PDX-1, as determined by real-time PCR in populations of the human embryonic stem cell line H1, at passage 48, differentiated into cells expressing markers characteristic of the pancreatic endoderm lineage, cultured on mouse embryonic fibroblasts (MEF), commercially available mouse embryonic fibroblasts (MEF-SM), human dermal fibroblasts (D551), human foreskin fibroblasts (Hs27), and human pancreatic-derived stromal cells (HP).

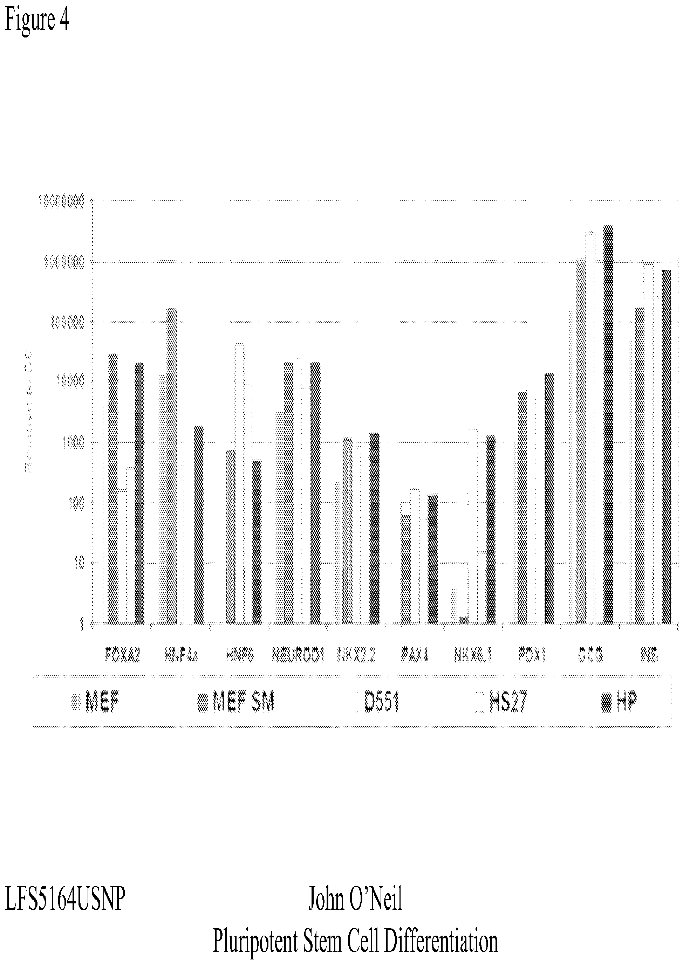

Figure 4 shows the effects of human feeder cell layers on the formation of cells expressing markers characteristic of the pancreatic endocrine lineage. The figure shows expression of FoxA2, HNF-4a, HNF-6, NeuroD1, Nkx 2.2, Pax-4, Nkx 6.1, PDX-1, glucagon (GCG), and insulin (INS), as determined by real-time PCR in populations of the human embryonic stem cell line H1, at passage 48, differentiated into cells expressing markers characteristic of the pancreatic endocrine lineage, cultured on mouse embryonic fibroblasts (MEF), commercially available mouse embryonic fibroblasts (MEF-SM), human dermal fibroblasts (D551), human foreskin fibroblasts (Hs27), and human pancreatic-derived stromal cells (HP).

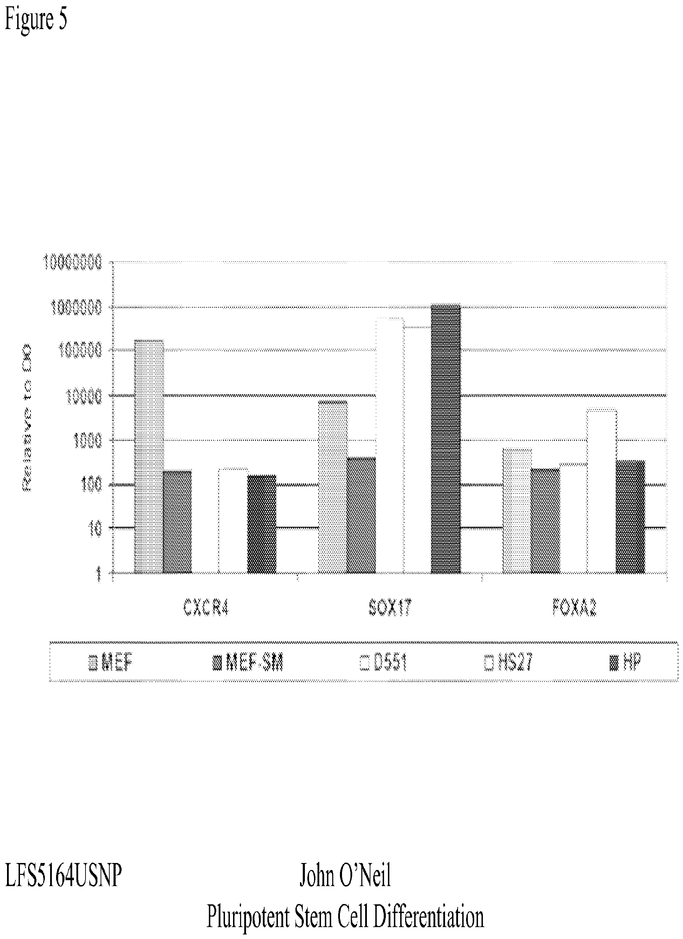

Figure 5 shows the effects of human feeder cell layers on the differentiation of human embryonic stem cells into cells expressing markers characteristic of the definitive endoderm lineage. The figure shows expression of CXCR4, Sox-17, and FoxA2, as determined by real-time PCR in populations of the human embryonic stem cell line H9, at passage 46, differentiated into cells expressing markers characteristic of the definitive endoderm lineage, cultured on mouse embryonic fibroblasts (MEF), commercially available mouse embryonic fibroblasts (MEF-SM), human dermal fibroblasts (D551), human foreskin fibroblasts (Hs27), and human pancreatic-derived stromal cells (HP).

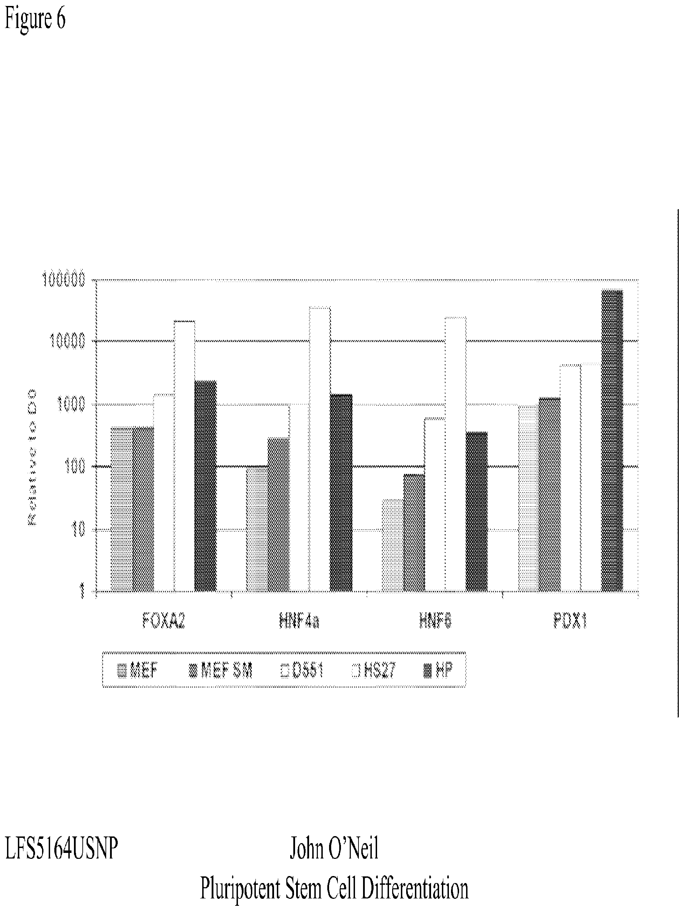

Figure 6 shows the effects of human feeder cell layers on the formation of cells expressing markers characteristic of the pancreatic endoderm lineage. The figure shows expression of FoxA2, HNF-4a, HNF-6 and PDX-1, as determined by real-time PCR in populations of the human embryonic stem cell line H9, at passage 46, differentiated into cells expressing markers characteristic of the pancreatic endoderm lineage, cultured on mouse embryonic fibroblasts (MEF), commercially available mouse embryonic fibroblasts (MEF-SM), human dermal fibroblasts (D551), human foreskin fibroblasts (Hs27), and human pancreatic-derived stromal cells (HP).

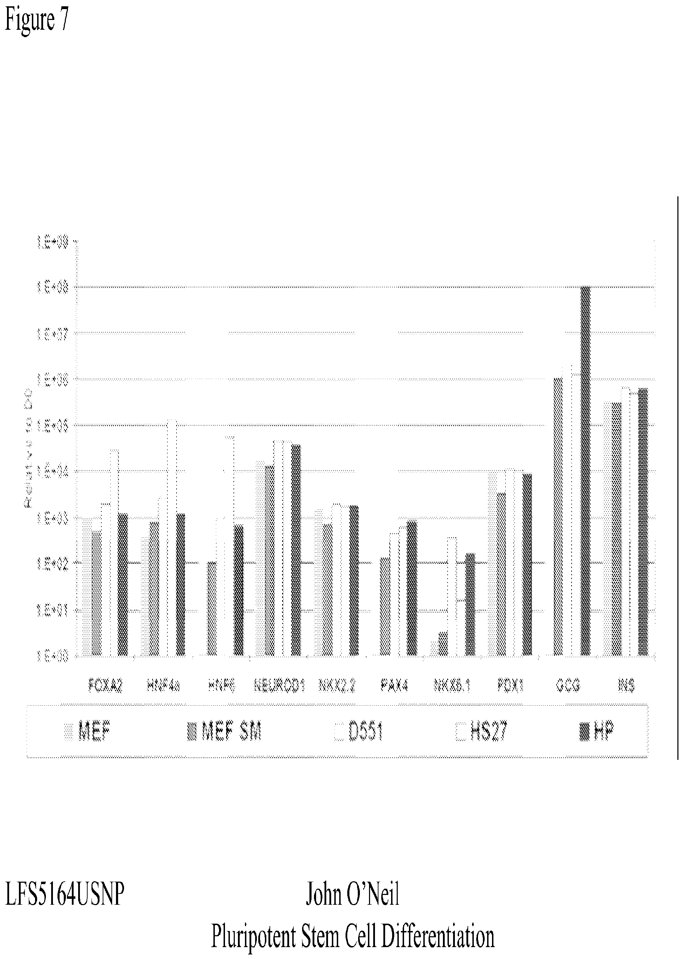

Figure 7 shows the effects of human feeder cell layers on the formation of cells expressing markers characteristic of the pancreatic endocrine lineage. The figure shows expression of FoxA2, HNF-4a, HNF-6, NeuroD1, Nkx 2.2, Pax-4, Nkx 6.1, PDX-1, glucagon (GCG), and insulin (INS), as determined by real-time PCR in populations of the human embryonic stem cell line H9, at passage 46, differentiated into cells expressing markers characteristic of the pancreatic endocrine lineage, cultured on mouse embryonic fibroblasts (MEF), commercially available mouse embryonic fibroblasts (MEF-SM), human dermal fibroblasts (D551), human foreskin fibroblasts (Hs27), and human pancreatic-derived stromal cells (HP).

### DETAILED DESCRIPTION

For clarity of disclosure, and not by way of limitation, the detailed description of the invention is divided into the following subsections that describe or illustrate certain features, embodiments or applications of the present invention.

### Definitions

Stem cells are undifferentiated cells defined by their ability at the single cell level to both self-renew and differentiate to produce progeny cells, including self-renewing progenitors, non-renewing progenitors, and terminally differentiated cells. Stem cells are also characterized by their ability to differentiate *in vitro* into functional cells of various cell lineages from multiple germ layers (endoderm, mesoderm and ectoderm), as well as to give rise to tissues of multiple germ layers following transplantation and to contribute substantially to most, if not all, tissues following injection into blastocysts.

Stem cells are classified by their developmental potential as: (1) totipotent, meaning able to give rise to all embryonic and extraembryonic cell types; (2) pluripotent, meaning able to give rise to all embryonic cell types; (3) multipotent, meaning able to give rise to a subset of cell lineages, but all within a particular tissue, organ, or physiological system (for example, hematopoietic stem cells (HSC) can produce progeny that include HSC (selfrenewal), blood cell restricted oligopotent progenitors and all cell types and elements (e.g., platelets) that are normal components of the blood); (4) oligopotent, meaning able to give rise to a more restricted subset of cell lineages than multipotent stem cells; and (5) unipotent, meaning able to give rise to a single cell lineage (e.g. , spermatogenic stem cells).

Differentiation is the process by which an unspecialized ("uncommitted") or less specialized cell acquires the features of a specialized cell such as, for example, a nerve cell or a muscle cell. A differentiated or differentiation-induced cell is one that has taken on a more specialized ("committed") position within the lineage of a cell. The term "committed", when applied to the process of differentiation, refers to a cell that has proceeded in the differentiation pathway to a point where, under normal circumstances, it will continue to differentiate into a specific cell type or subset of cell types, and cannot, under normal circumstances, differentiate into a different cell type or revert to a less differentiated cell type. De-differentiation refers to the process by which a cell reverts to a less specialized (or committed) position within the lineage of a cell. As used herein, the lineage of a cell defines the heredity of the cell, i.e., which cells it came from and what cells it can give rise to. The lineage of a cell places the cell within a hereditary scheme of development and differentiation. A lineage-specific marker refers to a characteristic specifically associated with the phenotype of cells of a lineage of interest and can be used to assess the differentiation of an uncommitted cell to the lineage of interest.

Various terms are used to describe cells in culture. "Maintenance" refers generally to cells placed in a growth medium under conditions that facilitate cell growth and/or division, which may or may not result in a larger population of the cells. "Passaging" refers to the process of removing the cells from one culture vessel and placing them in a second culture vessel under conditions that facilitate cell growth and/or division.

A specific population of cells, or a cell line, is sometimes referred to or characterized by the number of times it has been passaged. For example, a cultured cell population that has been passaged ten times may be referred to as a P10 culture. The primary culture, i.e., the first culture following the isolation of cells from tissue, is designated P0. Following the first subculture, the cells are described as a secondary culture (P1 or passage 1). After the second subculture, the cells become a tertiary culture (P2 or passage 2), and so on. It will be understood by those of skill in the art that there may be many population doublings during the period of passaging; therefore the number of population doublings of a culture is greater than the passage number. The expansion of cells (i.e., the number of population doublings) during the period between passages depends on many factors, including but not limited to the seeding density, substrate, medium, growth conditions, and time between passaging.

"β-cell lineage" refer to cells with positive gene expression for the transcription factor PDX-1 and at least one of the following transcription factors: NGN-3, Nkx2.2, Nkx6.1, NeuroD, Isl-1, HNF-3 beta, MAFA, Pax4, and Pax6. Cells expressing markers characteristic of the β cell lineage include β cells.

"Cells expressing markers characteristic of the definitive endoderm lineage" as used herein refer to cells expressing at least one of the following markers: SOX-17, GATA-4, HNF-3 beta, GSC, Cer1, Nodal, FGF8, Brachyury, Mixlike homeobox protein, FGF4 CD48, eomesodermin (EOMES), DKK4, FGF17, GATA-6, CXCR4, C-Kit, CD99, or OTX2. Cells expressing markers characteristic of the definitive endoderm lineage include primitive streak precursor cells, primitive streak cells, mesendoderm cells and definitive endoderm cells.

"Cells expressing markers characteristic of the pancreatic endoderm lineage" as used herein refer to cells expressing at least one of the following markers: PDX-1, HNF-1beta, HNF-3beta, PTF-1 alpha, HNF-6, or HB9. Cells expressing markers characteristic of the pancreatic endoderm lineage include pancreatic endoderm cells.

"Cells expressing markers characteristic of the pancreatic endocrine lineage" as used herein refer to cells expressing at least one of the following markers: NGN-3, NeuroD, Islet-1, PDX-1, NKX6.1, Pax-4, or PTF-1 alpha. Cells expressing markers characteristic of the pancreatic endocrine lineage include pancreatic endocrine cells, pancreatic hormone expressing cells, and pancreatic hormone secreting cells, and cells of the β-cell lineage.

"Definitive endoderm" as used herein refers to cells which bear the characteristics of cells arising from the epiblast during gastrulation and which form the gastrointestinal tract and its derivatives. Definitive endoderm cells express the following markers: CXCR4, HNF-3 beta, GATA-4, SOX-17, Cerberus, OTX2, goosecoid, c-Kit, CD99, and Mixl1.

"Extraembryonic endoderm" as used herein refers to a population of cells expressing at least one of the following markers: SOX-7, AFP, and SPARC.

"Markers" as used herein, are nucleic acid or polypeptide molecules that are differentially expressed in a cell of interest. In this context, differential expression means an increased level for a positive marker and a decreased level for a negative marker. The detectable level of the marker nucleic acid or polypeptide is sufficiently higher or lower in the cells of interest compared to other cells, such that the cell of interest can be identified and distinguished from other cells using any of a variety of methods known in the art.

"Mesendoderm cell" as used herein refers to a cell expressing at least one of the following markers: CD48, eomesodermin (EOMES), SOX-17, DKK4, HNF-3 beta, GSC, FGF17, GATA-6.

"Pancreatic endocrine cell" or "pancreatic hormone expressing cell" as used herein refers to a cell capable of expressing at least one of the following hormones: insulin, glucagon, somatostatin, pancreatic polypeptide and ghrelin.

"Pancreatic hormone secreting cell" as used herein refers to a cell capable of secreting at least one of the following hormones: insulin, glucagon, somatostatin, and pancreatic polypeptide.

"Pre-primitive streak cell" as used herein refers to a cell expressing at least one of the following markers: Nodal, or FGF8.

"Primitive streak cell" as used herein refers to a cell expressing at least one of the following markers: Brachyury, Mix-like homeobox protein, or FGF4.

The present invention relates to the field of pluripotent stem cell differentiation. The present invention provides methods for the propagation of pluripotent stem cells on a human feeder cell layer. The present invention also provides methods for the differentiation of pluripotent stem cells on a human feeder cell layer. In particular, the present invention provides an improved method for the differentiation of pluripotent stem cells into pancreatic endocrine cells using a human feeder cell layer.

In one embodiment, the present invention provides a method, according to the claims, for differentiating pluripotent stem cells, comprising the steps of:
a. Culturing the pluripotent stem cells,
b. Plating the pluripotent stem cells onto a human feeder cell layer, and
c. Treating the pluripotent stem cells with at least one factor that promotes the differentiation of the pluripotent stem cells.

The methods of the present invention provides an improved method for differentiating pluripotent stem cells, wherein the pluripotent stem cells are plated onto a human feeder cell layer prior to differentiating the pluripotent stem cells. The pluripotent stem cells may be cultured by any suitable method in the art. Likewise, the pluripotent stem cells may be plated onto the human feeder cell layer by any suitable method in the art. The pluripotent stem cells may be treated with at least one factor that promotes the differentiation of the pluripotent stem cells immediately after plating onto the human feeder cell layer. Alternatively, the pluripotent stem cells may be treated with at least one factor that promotes the differentiation of the pluripotent stem cells after the pluripotent stem cells have been cultured in the presence of the human feeder cell layer for a period of time. For example, the pluripotent stem cells may be cultured in the presence of the human feeder cell layer for a period of time sufficient for the pluripotent stem cells to form a monolayer.

Pluripotent stem cells may be plated onto the human feeder cell layer at any density. Optimal density however, may be depend on factors, such as, for example, the pluripotent stem cell used, the cells comprising the human feeder cell layer, the differentiated cell type, the size of the culture vessel, and the like. In one embodiment, the pluripotent stem cells are plated at a density such that the pluripotent stem cells are about 60% to about 80% confluent following 5 days of culture on the human feeder cell layer.

Pluripotent stem cells suitable for use in the present invention include, for example, cells that express at least one of the following markers characteristic of pluripotent cells: ABCG2, cripto, CD9, FoxD3, Connexin43, Connexin45, Oct4, Sox2, Nanog, hTERT, UTF-1, ZFP42, SSEA-3, SSEA-4, Tral-60, Tral-81.

The cells comprising the human feeder cell layer may be any human cell that is capable of promoting the differentiation of pluripotent stem cells. The cells comprising the human feeder cell layer may be adult cells. Alternatively, the cells comprising the human feeder cell layer may be fetal or embryonic. In one embodiment, the human feeder cell layer is comprised of fibroblast cells. In one embodiment, the fibroblast cells are dermal fibroblasts. The dermal fibroblasts may be the human dermal fibroblast cell line Detroit 551 (CCL-110 ATCC). In another embodiment, the fibroblasts cells are foreskin fibroblasts. The human foreskin fibroblast may be the human foreskin fibroblast line Hs27 (CRL-1634 ATCC).

Alternatively, the human feeder cell layer is comprised of pancreatic-derived stromal cells. In one embodiment, the pancreatic-derived stromal cells are the cells disclosed in US20040241761. In an alternate embodiment, the pancreatic-derived stromal cells are the cells disclosed in Science 306: 2261-2264, 2004. In an alternate embodiment, the pancreatic-derived stromal cells are the cells disclosed in Nature Biotechnology 22: 1115 - 1124, 2004. In an alternate embodiment, the pancreatic-derived stromal cells are the cells disclosed in US20030082155. In an alternate embodiment, the pancreatic-derived stromal cells are the cells disclosed in US5834308. In an alternate embodiment, the pancreatic-derived stromal cells are the cells disclosed in Proc Nat Acad Sci 97: 7999-8004, 2000. In an alternate embodiment, the pancreatic-derived stromal cells are the cells disclosed in WO2004011621. In an alternate embodiment, the pancreatic-derived stromal cells are the cells disclosed in WO03102134. In an alternate embodiment, the pancreatic-derived stromal cells are the cells disclosed in US2004015805. In an alternate embodiment, the pancreatic-derived stromal cells are the cells disclosed in US6458593. In an alternate embodiment, the pancreatic-derived stromal cells are the cells disclosed in WO2006094286. In an alternate embodiment, the pancreatic-derived stromal cells are of the H5f3P6 cell line that have been assigned ATCC No. PTA-6974.

### Generation of a Feeder Cell Layer

Human feeder cell layers described in this application are useful for differentiating pluripotent stem cells. It is recognized that other types of cells may benefit from being differentiated on these feeder cell layers, and the compositions of this invention may be used for such purposes without restriction.

In one aspect of the present invention, a feeder cell layer is generated by a method, according to the claims, which essentially involves:
a. Culturing the cells that will form the feeder layer, and
b. Inactivating the cells.

The cells that will form the feeder cell layer may be cultured on a suitable culture substrate. In one embodiment, the suitable culture substrate is an extracellular matrix component, such as, for example, those derived from basement membrane or that may form part of adhesion molecule receptor-ligand couplings. In one embodiment, a the suitable culture substrate is MATRIGEL® (Becton Dickenson). MATRIGEL® is a soluble preparation from Engelbreth-Holm-Swarm tumor cells that gels at room temperature to form a reconstituted basement membrane. In another embodiment, the suitable culture substrate is gelatin (Sigma).

Other extracellular matrix components and component mixtures are suitable as an alternative. Depending on the cell type being proliferated, this may include laminin, fibronectin, proteoglycan, entactin, heparan sulfate, and the like, alone or in various combinations.

The cells used to form the feeder cell layer may be inactivated (i.e., rendered incapable of substantial replication) by, for example, radiation, treatment with a chemical inactivator, such as, for example, mitomycin c, or by any other effective method.

The medium used for culturing the cells used to form the feeder cell layer can have any of several different formulae. The medium must be able to support the propagation of at least the cell line used to form the feeder cell layer. It is convenient that the medium also support the propagation of the pluripotent stem cells. However, as an alternative, the medium can be supplemented with other factors or otherwise processed to adapt it for propagating the pluripotent stem cells.

In one embodiment, the pancreatic-derived stromal cells are the cells disclosed in WO2006094286.

*Isolation of pancreatic*-*derived cells:* In one aspect of the present invention, pancreatic cells are isolated by a multi-stage method, which essentially involves:
a. Perfusion of a cadaver pancreas, living donor or autologous pancreas, with an enzymatic solution,
b. Mechanical dissociation of the perfused pancreas,
c. Layering the digested tissue over a polysucrose or Ficoll gradient, followed by centrifugation to yield three distinct interfaces,
d. Removing the tissues and cells at each interface,
e. Culturing the tissues and cells in standard tissue culture plates in a nutrient rich selection media containing less than 5% serum, and
f. Leaving the culture undisturbed for about 2 to 4 weeks without any media changes.

Perfusion of a cadaver pancreas can be achieved with any of the enzymatic solutions well known to those skilled in the art. An example of an enzymatic solution suitable for use in the present invention contains LIBERASE HI™ (Roche - 0.5 mg/ml) and DNase I (0.2 mg/ml).

Mechanical dissociation of the pancreatic tissue can be carried out rapidly by the use of a tissue processor. Alternatively, mechanical dissociation of the pancreatic tissue can be carried out using a Ricordi Chamber or other equivalent apparatus that enables a less destructive dissociation of the tissue, compared to other procedures.

The digested pancreatic tissues are then subjected to a polysucrose or Ficoll gradient centrifugation to yield three distinct interfaces, which are enriched in cells from islets, the ductal tissue and the acinar tissue, respectively. In one embodiment, the tissues and cells are removed from each interface and cultured separately. In an alternative embodiment, the tissues and cells from all the interfaces are combined and cultured. It has been determined in accordance with the present invention that pancreatic stromal cells can be derived from any of the three interfaces. Alternatively, a continuous gradient can be employed and the cell population of choice selected to generate the pancreatic stromal cells.

According to the present invention, the tissues and cells collected from one or more of the interfaces are cultured in a selection media to selectively enrich stromal cells in the cell population. The selection media is rich in nutrient and contains low levels of glucose and serum. Generally speaking, the selection media contains less than 5% serum, alternatively 1-3% serum, alternatively about 2% serum; and less than 30 mM glucose. In one embodiment, the selection media is supplemented with 2% serum that is derived from the same mammalian species that the donor pancreas was harvested from. Alternatively, fetal or calf serum, serum from other species, or other serum supplements or replacements can be used to supplement the selection media. An example of a suitable selection media is composed of DMEM (5 mM glucose), 2% fetal bovine serum (FBS), 100 U/µg penicillin/streptomycin, insulin-transferrin-selenium (ITS), 2 mM L-Glutamine, 0.0165 mM ZnSO₄, and 0.38 µM 2-mercaptoethanol.

During the culture in a selection media ("the selection phase"), the cells can be cultured under hypoxic or normoxic conditions. Under hypoxic conditions, oxygen levels are lower than 20%, alternatively lower than 10%, alternatively lower than 5%, but more than 1%.

Preferably, the culture should be maintained in the selection media undisturbed for about 2 to 4 weeks without any media changes, at which point the cells have typically become adherent to the culture substrate used. The selection phase is considered to be complete when there is no further increasein the number of adherent cells.

It has been discovered that the methods of tissue harvest and culturing in accordance with the present invention result in a cell population enriched in pancreatic stromal cells. By "enriched" is meant that pancreatic stromal cells account for at least about 30%, alternatively about 40%, alternatively about 50% of all the cells in the population.

Alternatively, the tissues and cells collected from one or more of the interfaces are cultured in a selection media to selectively enrich stromal cells in the cell population. The selection media is rich in nutrient and contains low levels of glucose. Generally speaking, the selection media contains less than 20% serum, alternatively 10 to 5% serum, alternatively about 10% serum; and less than 30 mM glucose. In one embodiment, the selection media is supplemented with 10% serum that is derived from the same mammalian species that the donor pancreas was harvested from. Alternatively, fetal or calf serum, serum from other species, or other serum supplements or replacements can be used to supplement the selection media. An example of a suitable selection media is composed of DMEM (5 mM glucose), 10% fetal bovine serum (FBS), 100 U/µg penicillin/streptomycin.

During the culture in a selection media ("the selection phase"), the cells can be cultured under hypoxic or normoxic conditions. Under hypoxic conditions, oxygen levels are lower than 20%, alternatively lower than 10%, alternatively lower than 5%, but more than 1%.

Under these culture conditions, the media is replaced regularly at 2-4 day intervals.

It has been discovered that the methods of tissue harvest and culturing in accordance with the present invention result in a cell population enriched in pancreatic stromal cells. By "enriched" is meant that pancreatic stromal cells account for at least about 30%, alternatively about 40%, or alternatively about 50% of all the cells in the population.

Subsequent to the initial phase of selection and cell attachment, the cells (enriched with stromal cells) are expanded under conditions as further described hereinbelow.

If desirable, the cell population enriched in stromal cells can be exposed, for example, to an agent (such as an antibody) that specifically recognizes a protein marker expressed by stromal cells, to identify and select pancreatic stromal cells, thereby obtaining a substantially pure population of pancreatic stromal cells.

*Characterization of the isolated pancreatic stromal cells:* Methods for assessing expression of protein and nucleic acid markers in cultured or isolated cells are standard in the art. These include quantitative reverse transcriptase polymerase chain reaction (RT-PCR), Northern blots, i hybridization (see, e.g., Current Protocols in Molecular Biology (Ausubel et al., eds. 2001 supplement)), and immunoassays, such as immunohistochemical analysis of sectioned material, Western blotting, and for markers that are accessible in intact cells, flow cytometry analysis (FACS) (see, *e.g*., Harlow and Lane, Using Antibodies: A Laboratory Manual, New York: Cold Spring Harbor Laboratory Press (1998)).

The pancreatic stromal cells isolated in accordance with the present invention are characterized as, inter alia, substantially lacking at least one of the following protein markers: CD117, NCAM, ABCG2, cytokeratin 7, 8, 18, or 19. In certain specific embodiments, the pancreatic stromal cells isolated in accordance with the present invention are characterized as substantially positive for at least one of the following protein markers: CD44, CD73, CD90 and CD105.

*Expansion of pancreatic stromal cells:* In a further aspect, the present invention provides a method for expanding the pancreatic stromal cells obtained in accordance with the present invention. As described hereinabove, pancreatic digests, which may contain a heterogeneous mixture of islets, ductal fragments and exocrine tissue, are cultured in a low serum selection media for 2-4 weeks, preferably without any media changes, to selectively enrich the desired stromal cells. The resulting cell population, now enriched with pancreatic stromal cells, is then switched to a growth media to expand the pancreatic stromal cells in the cell population.

The growth media suitable for use in the present invention can be composed of media such as DMEM containing penicillin/streptomycin (P/S) and serum at a concentration of 2% to 20%, alternatively about 5 to 10%. In one embodiment, the growth media is composed of DMEM (1000 mg/L D-glucose; 862 mg/L glutamine), and 10% fetal bovine serum. In an alternate embodiment, the growth media is supplemented with serum that is derived from the same mammalian species that the donor pancreas was harvested from. Alternatively, fetal or calf serum, or other serum supplements or replacements, such as, for example, serum albumin, may be used to supplement the growth media.

Furthermore, the stromal cells can be expanded by culturing in a defined growth media containing agent(s) that stimulate the proliferation of the cells of the present invention. These factors may include, for example, nicotinamide, members of TGF-β family, including TGF-β1, 2, and 3, bone morphogenic proteins (BMP-2, -4, 6, -7, -11, -12, and - 13), serum albumin, fibroblast growth factor family, platelet-derived growth factor-AA, and -BB, platelet rich plasma, insulin growth factor (IGF-I, II) growth differentiation factor (GDF-5, -6, -8, -10, 11), glucagon like peptide-I and II (GLP-I and II), GLP-1 and GLP-2 mimetobody, Exendin-4, retinoic acid, parathyroid hormone, insulin, progesterone, aprotinin, hydrocortisone, ethanolamine, beta mercaptoethanol, epidermal growth factor (EGF), gastrin I and II, copper chelators such as triethylene pentamine, TGF-α, forskolin, Na-Butyrate, activin, betacellulin, insulin/transferring/selenium (ITS), hepatocyte growth factor (HGF), keratinocyte growth factor (KGF), bovine pituitary extract, islet neogenesis-associated protein (INGAP), proteasome inhibitors, notch pathway inhibitors, sonic hedgehog inhibitors, or combinations thereof. Alternatively, the stromal cells may be expanded by culturing in conditioned media. By "conditioned media" is meant that a population of cells is grown in a basic defined cell culture medium and contributes soluble factors to the medium. In one such use, the cells are removed from the medium, while the soluble factors the cells produce remain. This medium is then used to nourish a different population of cells.

In certain embodiments, the pancreatic stromal cells are cultured on standard tissue culture plates. Alternatively, the culture plates may be coated with extracellular matrix proteins, such as, for example, MATRIGEL ®, growth factor reduced MATRIGEL ®, laminin, collagen, gelatin, tenascin, fibronectin, vitronectin, thrombospondin, placenta extracts or combinations thereof.

Furthermore, the pancreatic stromal cells can be expanded *in vitro* under hypoxic or normoxic conditions.

### Differentiation of Pluripotent Stem Cells

In one embodiment of the present invention, pluripotent stem cells are propagated in culture, while maintaining their pluripotency. Pluripotent stem cells are then transferred onto human feeder cell layers prior to differentiation. Changes in pluripotency of the cells with time can be determined by detecting changes in the levels of expression of markers associated with pluripotency. Alternatively, changes in pluripotency can be monitored by detecting changes in the levels of expression of markers associated with differentiation, or markers associated with another cell type.

The pluripotent cells are treated with at least one factor that promotes their differentiation into another cell type. The other cell type may be a cell expressing markers characteristic of the definitive endoderm lineage. Alternatively, the cell type may be a cell expressing markers characteristic of the pancreatic endoderm lineage. Alternatively, the cell type may be a cell expressing markers characteristic of the pancreatic endocrine lineage. Alternatively, the cell type may be a cell expressing markers characteristic of the β-cell lineage.

Pluripotent stem cells treated in accordance with the methods of the present invention may be differentiated into a variety of other cell types by any suitable method in the art. For example, pluripotent stem cells treated in accordance with the methods of the present invention may be differentiated into neural cells, cardiac cells, hepatocytes, and the like.

For example, pluripotent stem cells treated in accordance with the methods of the present invention may be differentiated into neural progenitors and cardiomyocytes according to the methods disclosed in WO2007030870.

In another example, pluripotent stem cells treated in accordance with the methods of the present invention may be differentiated into hepatocytes according to the methods disclosed in US patent 6,458,589.

### Differentiation of Pluripotent Stem Cells into Cells Expressing Markers Characteristic of the Pancreatic Endocrine Lineage

In one aspect of the present invention, cells expressing markers characteristic of the pancreatic endocrine linage are formed from pluripotent stem cells by a multistage method, according to the claims, comprising the steps of:
a. Culturing the pluripotent stem cells,
b. Plating the pluripotent cells on a human feeder cell layer,
c. Differentiating the pluripotent stem cells into cells expressing markers characteristics of the definitive endoderm lineage,
d. Differentiating the cells expressing markers characteristics of the definitive endoderm lineage into cells expressing markers characteristics of the pancreatic endoderm lineage, and
e. Differentiating the cells expressing markers characteristics of the pancreatic endoderm lineage into cells expressing markers characteristics of the pancreatic endocrine lineage.

Markers characteristic of the definitive endoderm lineage are selected from the group consisting of SOX-17, GATA4, Hnf-3beta, GSC, Cer1, Nodal, FGF8, Brachyury, Mix-like homeobox protein, FGF4 CD48, eomesodermin (EOMES), DKK4, FGF17, GATA6, CXCR4, C-Kit, CD99, and OTX2. Suitable for use in the present invention is a cell that expresses at least one of the markers characteristic of the definitive endoderm lineage. In one aspect of the present invention, a cell expressing markers characteristic of the definitive endoderm lineage is a primitive streak precursor cell. In an alternate aspect, a cell expressing markers characteristic of the definitive endoderm lineage is a mesendoderm cell. In an alternate aspect, a cell expressing markers characteristic of the definitive endoderm lineage is a definitive endoderm cell.

Markers characteristic of the pancreatic endoderm lineage are selected from the group consisting of Pdxl, HNF-1beta, PTF1a, HNF-6, HB9 and PROX1. Suitable for use in the present invention is a cell that expresses at least one of the markers characteristic of the pancreatic endoderm lineage. In one aspect of the present invention, a cell expressing markers characteristic of the pancreatic endoderm lineage is a pancreatic endoderm cell.

Markers characteristic of the pancreatic endocrine lineage are selected from the group consisting of NGN-3, NeuroD, Islet-1, Pdx-1, NKX6.1, Pax-4, and PTF-1 alpha. In one embodiment, a pancreatic endocrine cell is capable of expressing at least one of the following hormones: insulin, glucagon, somatostatin, and pancreatic polypeptide. Suitable for use in the present invention is a cell that expresses at least one of the markers characteristic of the pancreatic endocrine lineage. In one aspect of the present invention, a cell expressing markers characteristic of the pancreatic endocrine lineage is a pancreatic endocrine cell. The pancreatic endocrine cell may be a pancreatic hormone expressing cell. Alternatively, the pancreatic endocrine cell may be a pancreatic hormone secreting cell.

In one aspect of the present invention, the pancreatic endocrine cell is a cell expressing markers characteristic of the β cell lineage. A cell expressing markers characteristic of the β cell lineage expresses Pdxl and at least one of the following transcription factors: NGN-3, Nkx2.2, Nkx6.1, NeuroD, Isl-1, HNF-3 beta, MAFA, Pax4, and Pax6. In one aspect of the present invention, a cell expressing markers characteristic of the β cell lineage is a β cell.

For example, pluripotent stem cells may be differentiated into cells expressing markers characteristic of the pancreatic endocrine lineage according to the methods disclosed in D'Amour et al, Nature Biotechnology 24, 1392 - 1401 (2006).

*Formation of cells expressing markers characteristic of the definitive endoderm lineage:* Pluripotent stem cells may be differentiated into cells expressing markers characteristic of the definitive endoderm lineage by any method in the art or by any method proposed in this invention.

For example, pluripotent stem cells may be differentiated into cells expressing markers characteristic of the definitive endoderm lineage according to the methods disclosed in D'Amour et al, Nature Biotechnology 23, 1534 - 1541 (2005).

For example, pluripotent stem cells may be differentiated into cells expressing markers characteristic of the definitive endoderm lineage according to the methods disclosed in Shinozaki et al, Development 131, 1651 - 1662 (2004).

For example, pluripotent stem cells may be differentiated into cells expressing markers characteristic of the definitive endoderm lineage according to the methods disclosed in McLean et al, Stem Cells 25, 29 - 38 (2007).

For example, pluripotent stem cells may be differentiated into cells expressing markers characteristic of the definitive endoderm lineage according to the methods disclosed in D'Amour et al, Nature Biotechnology 24, 1392 - 1401 (2006).

For example, pluripotent stem cells may be differentiated into cells expressing markers characteristic of the definitive endoderm lineage by culturing the pluripotent stem cells in medium containing activin A in the absence of serum, then culturing the cells with activin A and serum, and then culturing the cells with activin A and serum of a different concentration. An example of this method is disclosed in Nature Biotechnology 23, 1534 - 1541 (2005).

For example, pluripotent stem cells may be differentiated into cells expressing markers characteristic of the definitive endoderm lineage by culturing the pluripotent stem cells in medium containing activin A in the absence of serum, then culturing the cells with activin A with serum of another concentration. An example of this method is disclosed in D' Amour et al, Nature Biotechnology, 2005.

For example, pluripotent stem cells may be differentiated into cells expressing markers characteristic of the definitive endoderm lineage by culturing the pluripotent stem cells in medium containing activin A and a Wnt ligand in the absence of serum, then removing the Wnt ligand and culturing the cells with activin A with serum. An example of this method is disclosed in Nature Biotechnology 24, 1392 - 1401 (2006).

*Formation of cells expressing markers characteristic of the pancreatic endoderm lineage:* Cells expressing markers characteristic of the definitive endoderm lineage may be differentiated into cells expressing markers characteristic of the pancreatic endoderm lineage by any method in the art or by any method proposed in this invention.

For example, cells expressing markers characteristic of the definitive endoderm lineage may be differentiated into cells expressing markers characteristic of the pancreatic endoderm lineage according to the methods disclosed in D'Amour et al, Nature Biotechnology 24, 1392 - 1401 (2006).

For example, cells expressing markers characteristic of the definitive endoderm lineage are further differentiated into cells expressing markers characteristic of the pancreatic endoderm lineage, by treating the cells expressing markers characteristic of the definitive endoderm lineage with a fibroblast growth factor and the hedgehog signaling pathway inhibitor KAAD-cyclopamine, then removing the medium containing the fibroblast growth factor and KAAD-cyclopamine and subsequently culturing the cells in medium containing retinoic acid, a fibroblast growth factor and KAAD-cyclopamine. An example of this method is disclosed in Nature Biotechnology 24, 1392 - 1401 (2006).

*Formation of cells expressing markers of the pancreatic endocrine lineage:* Cells expressing markers characteristic of the pancreatic endoderm lineage may be differentiated into cells expressing markers characteristic of the pancreatic endocrine lineage by any method in the art or by any method disclosed in this invention.

For example, cells expressing markers characteristic of the pancreatic endoderm lineage may be differentiated into cells expressing markers characteristic of the pancreatic endocrine lineage according to the methods disclosed in D'Amour et al, Nature Biotechnology 24, 1392 - 1401 (2006).

For example, cells expressing markers characteristic of the pancreatic endoderm lineage are further differentiated into cells expressing markers characteristic of the pancreatic endocrine lineage, by culturing the cells expressing markers characteristic of the pancreatic endoderm lineage in medium containing DAPT and exendin 4, then removing the medium containing DAPT and exendin 4 and subsequently culturing the cells in medium containing exendin 1, IGF-1 and HGF. An example of this method is disclosed in Nature Biotechnology 24, 1392 - 1401 (2006).

For example, cells expressing markers characteristic of the pancreatic endoderm lineage are further differentiated into cells expressing markers characteristic of the pancreatic endocrine lineage, by culturing the cells expressing markers characteristic of the pancreatic endoderm lineage in medium containing exendin 4, then removing the medium containing exendin 4 and subsequently culturing the cells in medium containing exendin 4, IGF-1 and HGF. An example of this method is disclosed in D' Amour et al, Nature Biotechnology, 2006.

For example, cells expressing markers characteristic of the pancreatic endoderm lineage are further differentiated into cells expressing markers characteristic of the pancreatic endocrine lineage, by culturing the cells expressing markers characteristic of the pancreatic endoderm lineage in medium containing DAPT and exendin 4. An example of this method is disclosed in D' Amour et al, Nature Biotechnology, 2006.

For example, cells expressing markers characteristic of the pancreatic endoderm lineage are further differentiated into cells expressing markers characteristic of the pancreatic endocrine lineage, by culturing the cells expressing markers characteristic of the pancreatic endoderm lineage in medium containing exendin 4. An example of this method is disclosed in D' Amour et al, Nature Biotechnology, 2006.

### Isolation, Expansion and Culture of Pluripotent Stem Cells

### Characterization of Pluripotent Stem Cells

Pluripotent stem cells may express one or more of the stage-specific embryonic antigens (SSEA) 3 and 4, and markers detectable using antibodies designated Tra-1-60 and Tra-1-81 (Thomson et al., Science 282:1145, 1998). Differentiation of pluripotent stem cells *in vitro* results in the loss of SSEA-4, Tra- 1-60, and Tra-1-81 expression (if present) and increased expression of SSEA-1. Undifferentiated pluripotent stem cells typically have alkaline phosphatase activity, which can be detected by fixing the cells with 4% paraformaldehyde, and then developing with Vector Red as a substrate, as described by the manufacturer (Vector Laboratories, Burlingame Calif.). Undifferentiated pluripotent stem cells also typically express Oct-4 and TERT, as detected by real time PCR.

Another desirable phenotype of propagated pluripotent stem cells is a potential to differentiate into cells of all three germinal layers: endoderm, mesoderm, and ectoderm tissues. Pluripotency of pluripotent stem cells can be confirmed, for example, by injecting cells into severe combined immunodeficient (SCID) mice, fixing the teratomas that form using a fixative such as 4% paraformaldehyde, and then examining them histologically for evidence of cell types from the three germ layers. Alternatively, pluripotency may be determined by the creation of embryoid bodies and assessing the embryoid bodies for the presence of markers associated with the three germinal layers.

Propagated pluripotent stem cell lines may be karyotyped using a standard G-banding technique and compared to published karyotypes of the corresponding primate species. It is desirable to obtain cells that have a "normal karyotype," which means that the cells are euploid, wherein all human chromosomes are present and not noticeably altered.

### Culture of pluripotent Stem Cells

In one embodiment, pluripotent stem cells are typically cultured on a layer of feeder cells that support the pluripotent stem cells in various ways. Alternatively, pluripotent stem cells are cultured in a culture system that is essentially free of feeder cells, but nonetheless supports proliferation of pluripotent stem cells without undergoing substantial differentiation. The growth of pluripotent stem cells in feeder-free culture without differentiation is supported using a medium conditioned by culturing previously with another cell type. Alternatively, the growth of pluripotent stem cells in feeder-free culture without differentiation is supported using a chemically defined medium.

For example, Reubinoff et al (Nature Biotechnology 18: 399 - 404 (2000)) and Thompson et al (Science 6 November 1998: Vol. 282. no. 5391, pp. 1145-1147) disclose the culture of pluripotent stem cell lines from human blastocysts using a mouse embryonic fibroblast feeder cell layer.

Richards et al, (Stem Cells 21: 546-556, 2003) evaluated a panel of 11 different human adult, fetal and neonatal feeder cell layers for their ability to support human pluripotent stem cell culture. Richards *et al,* states: "human embryonic stem cell lines cultured on adult skin fibroblast feeders retain human embryonic stem cell morphology and remain pluripotent".

US20020072117 discloses cell lines that produce media that support the growth of primate pluripotent stem cells in feeder-free culture. The cell lines employed are mesenchymal and fibroblast-like cell lines obtained from embryonic tissue or differentiated from embryonic stem cells. US20020072117 also discloses the use of the cell lines as a primary feeder cell layer.

In another example, Wang et al (Stem Cells 23: 1221-1227, 2005) discloses methods for the long-term growth of human pluripotent stem cells on feeder cell layers derived from human embryonic stem cells.

In another example, Stojkovic et al (Stem Cells 2005 23: 306-314, 2005) disclose a feeder cell system derived from the spontaneous differentiation of human embryonic stem cells.

In a further example, Miyamoto et al (Stem Cells 22: 433-440, 2004) disclose a source of feeder cells obtained from human placenta.

Amit et al (Biol. Reprod 68: 2150-2156, 2003) discloses a feeder cell layer derived from human foreskin.

In another example, Inzunza et al (Stem Cells 23: 544-549, 2005) disclose a feeder cell layer from human postnatal foreskin fibroblasts.

US6642048 discloses media that support the growth of primate pluripotent stem (pPS) cells in feeder-free culture, and cell lines useful for production of such media. US6642048 states: "This invention includes mesenchymal and fibroblast-like cell lines obtained from embryonic tissue or differentiated from embryonic stem cells. Methods for deriving such cell lines, processing media, and growing stem cells using the conditioned media are described and illustrated in this disclosure."

In another example, WO2005014799 discloses conditioned medium for the maintenance, proliferation and differentiation of mammalian cells. WO2005014799 states: "The culture medium produced in accordance with the present invention is conditioned by the cell secretion activity of murine cells, in particular, those differentiated and immortalized transgenic hepatocytes, named MMH (Met Murine Hepatocyte)."

In another example, Xu et al (Stem Cells 22: 972-980, 2004) discloses conditioned medium obtained from human embryonic stem cell derivatives that have been genetically modified to over express human telomerase reverse transcriptase.

In another example, US20070010011 discloses a chemically defined culture medium for the maintenance of pluripotent stem cells.

An alternative culture system employs serum-free medium supplemented with growth factors capable of promoting the proliferation of embryonic stem cells. For example, Cheon et al (BioReprod DOI:10.1095/biolreprod.105.046870, October 19, 2005) disclose a feeder-free, serum-free culture system in which embryonic stem cells are maintained in unconditioned serum replacement (SR) medium supplemented with different growth factors capable of triggering embryonic stem cell self-renewal.

In another example, Levenstein et al (Stem Cells 24: 568-574, 2006) disclose methods for the long-term culture of human embryonic stem cells in the absence of fibroblasts or conditioned medium, using media supplemented with bFGF.

In another example, US20050148070 discloses a method of culturing human embryonic stem cells in defined media without serum and without fibroblast feeder cells, the method comprising: culturing the stem cells in a culture medium containing albumin, amino acids, vitamins, minerals, at least one transferrin or transferrin substitute, at least one insulin or insulin substitute, the culture medium essentially free of mammalian fetal serum and containing at least about 100 ng/ml of a fibroblast growth factor capable of activating a fibroblast growth factor signaling receptor, wherein the growth factor is supplied from a source other than just a fibroblast feeder layer, the medium supported the proliferation of stem cells in an undifferentiated state without feeder cells or conditioned medium.

In another example, US20050233446 discloses a defined media useful in culturing stem cells, including undifferentiated primate primordial stem cells. In solution, the media is substantially isotonic as compared to the stem cells being cultured. In a given culture, the particular medium comprises a base medium and an amount of each of bFGF, insulin, and ascorbic acid necessary to support substantially undifferentiated growth of the primordial stem cells.

In another example, US6800480 states "In one embodiment, a cell culture medium for growing primate-derived primordial stem cells in a substantially undifferentiated state is provided which includes a low osmotic pressure, low endotoxin basic medium that is effective to support the growth of primate-derived primordial stem cells. The basic medium is combined with a nutrient serum effective to support the growth of primate-derived primordial stem cells and a substrate selected from the group consisting of feeder cells and an extracellular matrix component derived from feeder cells. The medium further includes non-essential amino acids, an anti-oxidant, and a first growth factor selected from the group consisting of nucleosides and a pyruvate salt."

In another example, US20050244962 states: "In one aspect the invention provides a method of culturing primate embryonic stem cells. One cultures the stem cells in a culture essentially free of mammalian fetal serum (preferably also essentially free of any animal serum) and in the presence of fibroblast growth factor that is supplied from a source other than just a fibroblast feeder layer. In a preferred form, the fibroblast feeder layer, previously required to sustain a stem cell culture, is rendered unnecessary by the addition of sufficient fibroblast growth factor."

In a further example, WO2005065354 discloses a defined, isotonic culture medium that is essentially feeder-free and serum-free, comprising: a. a basal medium; b. an amount of bFGF sufficient to support growth of substantially undifferentiated mammalian stem cells; c. an amount of insulin sufficient to support growth of substantially undifferentiated mammalian stem cells; and d. an amount of ascorbic acid sufficient to support growth of substantially undifferentiated mammalian stem cells.

In another example, WO2005086845 discloses a method for maintenance of an undifferentiated stem cell, said method comprising exposing a stem cell to a member of the transforming growth factor-beta (TGFβ) family of proteins, a member of the fibroblast growth factor (FGF) family of proteins, or nicotinamide (NIC) in an amount sufficient to maintain the cell in an undifferentiated state for a sufficient amount of time to achieve a desired result.

The pluripotent stem cells may be plated onto a suitable culture substrate. In one embodiment, the suitable culture substrate is an extracellular matrix component, such as, for example, those derived from basement membrane or that may form part of adhesion molecule receptor-ligand couplings. In one embodiment, a suitable culture substrate is MATRIGEL® (Becton Dickenson). MATRIGEL® is a soluble preparation from Engelbreth-Holm-Swarm tumor cells that gels at room temperature to form a reconstituted basement membrane.

Other extracellular matrix components and component mixtures are suitable as an alternative. Depending on the cell type being proliferated, this may include laminin, fibronectin, gelatin, proteoglycan, entactin, heparan sulfate, and the like, alone or in various combinations.

The pluripotent stem cells may be plated onto the substrate in a suitable distribution and in the presence of a medium that promotes cell survival, propagation, and retention of the desirable characteristics. All these characteristics benefit from careful attention to the seeding distribution and can readily be determined by one of skill in the art.

Suitable culture media may be made from the following components, such as, for example, Dulbecco's modified Eagle's medium (DMEM), Gibco # 11965-092; Knockout Dulbecco's modified Eagle's medium (KO DMEM), Gibco #10829-018; Ham's F12/50% DMEM basal medium; 200 mM L-glutamine, Gibco # 15039-027; non-essential amino acid solution, Gibco 11140-050; β-mercaptoethanol, Sigma # M7522; human recombinant basic fibroblast growth factor (bFGF), Gibco # 13256-029.

The present invention is further illustrated, but not limited by, the following examples.

### EXAMPLES

### Example 1

### The Establishment of Human Pancreatic Cell Lines

*Pancreas Preparation* - Human pancreata not suitable for clinical transplantation were obtained from The National Disease Research Interchange (Philadelphia, PA), following appropriate consent for research use. The pancreas was transferred with organ preservation solution to a stainless steel pan on ice and trimmed of all extraneous tissue. The pancreatic duct was cannulated with an 18 gauge catheter and the pancreas was injected with an enzyme solution, which contained the LIBERASE HI ™ enzyme (Roche - 0.5 mg/ml) and DNase I (0.2 mg/ml), dissolved in Dulbecco's Phosphate Buffered Saline (DPBS).

*Rapid Mechanical Dissociation Followed by Enzymatic Digestion* - The enzyme infused pancreata were homogenized in a tissue processor, pulsed 3-5 times for 3-5 seconds/pulse, and the dissociated tissue were transferred to two 500 ml trypsinizing flasks (Bellco) containing magnetic stir bars. Thereafter, 50-100 ml of the enzyme solution was added to each flask. The flasks were placed in a 37°C waterbath on submersible stir plates and allowed to incubate with an intermediate stir rate for 10 minutes. The stirring was stopped and the fine digested tissue was removed from the flask and transferred into 250 ml tube containing DPBS, 5% Fetal Bovine Serum (FBS) and 0.1 mg/ml DNase I (DPBS+) at 4°C to quench the digestion process. The flasks were replenished with 50-100 ml of the enzyme solution and returned to the waterbath and the stirring was re-initiated for an additional ten minutes. Again, the flasks were removed and the fine digest was collected and transferred to the 250 ml tubes on ice. This process was repeated for additional 3-5 times until the pancreas was completely digested.

*Gradual Mechanical Dissociation with Simultaneous Enzyme Digestion* - The enzyme infused pancreata were processed according to methods as described in Diabetes 37:413-420 (1988). Briefly, the pancreata were cleaned of extraneous tissue and injected with the enzyme solution as described above. The pancreata were then placed into a Ricordi Chamber with beads and covered with a screen with a mesh size of 400-600 µm to retain larger clusters of tissue. The chamber was covered and the enzyme solution was circulated through the chamber at approximately 37°C and the chamber was shaken to allow beads to disrupt pancreatic tissue while the enzyme digested the pancreas. Once adequate dissociation and digestion was achieved, the digestion was terminated and the tissue was collected.

*Tissue Separation -* The collected tissue was centrifuged at 150 x g for 5 minutes at 4°C. The supernatant was aspirated and the tissue was washed two additional times in DPBS+. Following the final wash, the tissue was applied to a discontinuous gradient for purification. The digested tissue was suspended in polysucrose (Mediatech, VA) with a density of 1.108 g/ml at a ratio of 1-2 ml tissue pellet per 10 ml of polysucrose solution. The tissue suspension was then transferred to round-bottom polycarbonate centrifuge tubes and polysucrose solutions with densities of 1.096 and 1.037 were carefully applied to the tubes. A final layer of DMEM completed the discontinuous purification gradient. The gradient tubes were centrifuged at 2000 rpm for 20 minutes at 4°C with no brake applied. Following centrifugation, the tissue was collected from each interface (three interfaces) and washed several times in DPBS+ as described above and collected in a 50 ml test tube.

*Further Cell Cluster Dissociation -* Optionally, one can further dissociate large cell clusters obtained using the above protocol into smaller clusters or single cell suspensions. After the final wash, the tissue from each fraction was suspended in 10 ml 1X trypsin/EDTA solution containing 200U/ml DNase I. The tubes were placed in the water bath and repeatedly aspirated and discharged from a 10 ml serological pipette for 5-6 minutes until a near single cell suspension is achieved. The digestion was quenched with the addition of 4°C DPBS+ and the tubes centrifuged at 800 rpm for 5 minutes. The cell suspensions were washed with DPBS+ and cultured as described below.

*Pancreatic Cell Culture -* Following the final wash, the cells from each interface were resuspended in DMEM, 2% FBS, 100 U/ µg penicillin/streptomycin, ITS, 2 mM L-Glutamine, 0.0165 mM ZnSO4 (Sigma), and 0.38 µM 2-mercaptoethanol (Invitrogen, CA) (hereinafter "the selection media"). Six ml of the cell suspension was seeded in T-25 tissue culture flasks and 12 ml of the cell suspension was seeded into T-75 flasks. The flasks were placed in 37°C incubators with 5% CO₂. Following 2-4 weeks culture, a complete media change was performed and adherent cells were returned to culture in DMEM (2750 mg/L D-glucose, 862 mg/L glutamine) (Gibco, CA) with 5% FBS (HyClone, UT), 1% P/S, 0.0165 mM ZnSO4 (hereinafter "the growth media") and allowed to reach near confluence (this stage is referred to as "passage 0" or "P0"), at which point they were passaged. Subsequent culturing of the cells was at 5000 cell/cm² in the growth media. Cultures were passaged every 7-10 days at approximately 70-90% confluency. It was shown that stromal cells were isolated from each of the three fractions present following the purification gradient.

### Example 2

### Culturing of Stromal Pancreatic Cells

Pancreatic cells isolated according to Example 1 were either cultured under hypoxic conditions (5% CO₂, 3% O₂, and 92% N₂) or normoxic conditions (5% CO₂, 20% O₂, and 75% N₂) for 2-4 weeks in the selection media. The cultures were then switched to the growth media and fed two to three times per week. After the initial culture period, adherent cells were observed in plates cultured under hypoxic and normoxic conditions. Furthermore, following the initial 2-4 wks of culturing, there were very few remaining islet-like or ductal structures in the plates.

### Reference example 3

### Expression of Genes Associated with Differentiation in Pluripotent Stem Cells Cultured on Feeder Cells Layers

The human embryonic stem cell line H9 was obtained from WiCell Research Institute, Inc., (Madison, WI) and cultured according to instructions provided by the source institute. Undifferentiated H9 human embryonic stem cells that had been maintained on inactivated primary mouse embryonic fibroblasts (MEF) were cryopreserved in 60% FBS, 20% DMSO, and 20% DMEM/F12 (Invitrogen/GIBCO) supplemented with 20% knockout serum replacement, 100 nM MEM nonessential amino acids, 0.5 mM beta-mercaptoethanol, 2mM L-glutamine with 4ng/ml human basic fibroblast growth factor (bFGF) (all from Invitrogen/GIBCO) at a rate of -1 °C/min and stored in vapor nitrogen. The cells were thawed and plated onto mitocycin c treated D551 human dermal fibroblasts seeded at a density of 52,000 cell/ cm². Following three passages on the D551 cells, the pluripotent cells were harvested and then transferred on MATRIGEL in conditioned medium from cultures of inactivated MEF supplemented with 8 ng/ml bFGF. Human embryonic stem cells plated on MATRIGEL (1:30) were cultured at 37°C in an atmosphere of 5% CO₂ within a humidified tissue culture incubator in 60 mm tissue culture plates. When confluent (approximately 5-7 days after plating), human embryonic stem cells were treated with 1mg/ml dispase (Invitrogen/GIBCO) for 25-40 min. Once the cells were released from the plate, they were pipted repeatedly with a 2 ml serological pipet until the desired colony size was achieved. Cells were spun at 1000 rpm for 5 min, and the pellet was resuspended and plated at a 1:3 to 1:4 ratio of cells in fresh culture medium onto MATRIGEL coated cell culture plates. Following five to ten passages on MATRIGEL, the pluripotent H9 cells were transferred to a number of different feeder cells. Briefly, cells were cultured on the feeders in ES cell medium consisting of DMEM/F12 (Invitrogen/GIBCO) supplemented with 20% knockout serum replacement, 100 nM MEM nonessential amino acids, 0.5 mM beta-mercaptoethanol, 2mM L-glutamine with 4ng/ml human basic fibroblast growth factor (bFGF) (all from Invitrogen/GIBCO) in tissue culture treated 6 well plates. The plates are prepared by coating with 0.1% gelatin (Sigma) and incubating at 37°C for a minimum of 4 hrs prior to seeding the feeders. Just prior to seeding the gelatin is aspirated and the feeder cell suspension delivered to each well of the 6 well plate. The cells were allowed to expand for 5 days prior to initiating the differentiation protocol.

The ability of the human dermal fibroblast cell line D551 (ATCC No. CCL-110), the human foreskin fibroblast cell line Hs27 (ATCC No. CRL-1634), and the human pancreatic-derived stromal cell line (disclosed in WO2006094286) to maintain pluripotency were evaluated. The D551 human feeder cells were cultured in EMEM (ATCC 30-2003) supplemented with 10% FBS. Once confluent, the cells were inactivated by mitomycin-C treatment and cryopreserved in EMEM, 10% FBS, and 5% DMSO at a rate of -1 °C/min and stored in vapor nitrogen. The cells were thawed at 37°C and seeded onto gelatin-coated tissue culture plates at 52,000/cm² in EMEM with 10% FBS. The Hs27 human feeder cells were cultured in DMEM (ATCC 30-2002) supplemented with 10% FBS. Once confluent, the cells were inactivated by mitomycin-C treatment and cryopreserved in DMEM, 10% FBS, and 5% DMSO at a rate of - 1°C/min and stored in vapor nitrogen. The cells were thawed at 37°C and seeded onto gelatin-coated tissue culture plates at 55,000/cm² in DMEM with 10% FBS. The human pancreatic-derived stromal cell line were cultured in DMEM and 10% FBS until confluent and treated with mitomycin C. The cells were cryopreserved in 90% FBS and 10% DMSO at a rate of -1°C/min and stored in vapor nitrogen. The cells were thawed at 37°C and seeded onto gelatin-coated tissue culture plates at 43,000/cm² in DMEM with 10% FBS. Cultures of human embryonic stem cells, plated onto commercially available mouse embryonic fibroblasts (MEFSM), and freshly derived mouse embryonic fibroblasts (MEF) were included as controls.

Plates of inactivated human feeder cells were washed with PBS and seeded with embryonic stem cells in ES medium. Embryonic stem cells were cultured on the human feeder cell layers for 5 days. After this time, the expression of CXCR4, Sox-17, Fox-A2, HNF-4a, HNF-6 and AFP was determined by real-time PCR from human embryonic stem cells cultured on mouse embryonic fibroblasts (MEF, Figure 1), commercially available mouse embryonic fibroblasts (MEF-SM, Figure 1), human dermal fibroblasts (D551, Figure 1), human foreskin fibroblasts (Hs27, Figure 1), and the human pancreatic-derived stromal cell line disclosed in WO2006094286 (HP, Figure 1). Results from a representative experiment are shown in Figure 1. Results were normalized to human embryonic stem cells cultured on MATRIGEL (Off MG, Figure 1). CXCR4, Sox-17, Fox-A2, HNF-4a, HNF-6 and AFP are markers associated with differentiation. Culture of human embryonic stem cells on the human feeder cell layer resulted in the decrease in expression of these markers. These data suggest that the human dermal fibroblast cell line D551, human foreskin fibroblasts Hs27, and the human pancreatic-derived stromal cell line disclosed in WO2006094286 maintain the pluripotency of human embryonic stem cells.

### Reference example 4

### Differentiation of Human Embryonic Stem Cells into Cells Expressing Markers Characteristic of the Pancreatic Endocrine Lineage on Human Feeder Cell Layers.

The human embryonic stem cell lines H1 and H9 was obtained from WiCell Research Institute, Inc., (Madison, WI) and cultured according to instructions provided by the source institute. Undifferentiated H1 & H9 human embryonic stem cells that had been maintained on inactivated primary mouse embryonic fibroblasts (MEF) were cryopreserved in 60% FBS, 20% DMSO, and 20% DMEM/F12 (Invitrogen/GIBCO) supplemented with 20% knockout serum replacement, 100 nM MEM nonessential amino acids, 0.5 mM betamercaptoethanol, 2mM L-glutamine with 4ng/ml human basic fibroblast growth factor (bFGF) (all from Invitrogen/GIBCO) at a rate of -1 °C/min and stored in vapor nitrogen. The cells were thawed and plated onto mitocycin C treated D551 human dermal fibroblasts seeded at a density of 52,000 cell/ cm². Following three passages on the D551 cells, the pluripotent cells were harvested and then transferred on MATRIGEL in conditioned medium from cultures of inactivated MEF supplemented with 8 ng/ml bFGF. Human embryonic stem cells plated on MATRIGEL (1:30) were cultured at 37°C in an atmosphere of 5% CO₂ within a humidified tissue culture incubator in 60 mm tissue culture plates. When confluent (approximately 5-7 days after plating), human embryonic stem cells were treated with 1mg/ml dispase (Invitrogen/GIBCO) for 25-40 min. Once the cells were released from the plate, they were pipted repeatedly with a 2 ml serological pipet until the desired colony size was achieved. Cells were spun at 1000 rpm for 5 min, and the pellet was resuspended and plated at a 1:3 to 1:4 ratio of cells in fresh culture medium onto MATRIGEL coated cell culture plates. Following eleven passages on MATRIGEL, the pluripotent H1 & H9 cells were transferred to a number of different feeder cells described below. Briefly, cells were cultured on the feeders in ES cell medium consisting of DMEM/F12 (Invitrogen/GIBCO) supplemented with 20% knockout serum replacement, 100 nM MEM nonessential amino acids, 0.5 mM beta-mercaptoethanol, 2mM L-glutamine with 4ng/ml human basic fibroblast growth factor (bFGF) (all from Invitrogen/GIBCO) in tissue culture treated 6 well plates. The plates are prepared by coating with 0.1 % gelatin (Sigma) and incubating at 37°C for a minimum of 4 hrs prior to seeding the feeders. Just prior to seeding the gelatin is aspirated and the feeder cell suspension delivered to each well of the 6 well plate. The cells were allowed to expand for 5 days prior to initiating the differentiation protocol.

The ability of human feeder cell layers to support the differentiation of human embryonic stem cells was evaluated. Embryonic stem cells were cultured on the mitomycin C inactivated human feeder cell layers for 5 days. Cultures of human embryonic stem cells, plated onto commercially available mouse embryonic fibroblasts (MEF-SM), and freshly derived mouse embryonic fibroblasts (MEF) were included as controls.

The ability of human dermal fibroblasts (D551, Figures 2 and 5), human foreskin fibroblasts (HS27, Figures 2 and 5), and the human pancreatic-derived stromal cell line disclosed in WO2006094286 (HP, Figures 2 and 5) to support the differentiation of populations of the human embryonic stem cell line H9 (Figure 2) and H1 (Figure 5) into cells expressing markers characteristic of the definitive endoderm lineage was evaluated. Populations of embryonic stem cells cultured on commercially available mouse embryonic fibroblasts (MEF-SM, Figures 2 and 5), and freshly derived mouse embryonic fibroblasts (MEF, Figures 2 and 5) were included as controls. Activin A (100ng/ml) was added to populations of human embryonic stem cells cultured on the feeder cell layers. Cells were cultured continuously in the presence of activin A and harvested after 3 days. The level of expression of markers characteristic of the definitive endoderm lineage were analyzed by real-time PCR (Figures 2 and 5). Results shown in Figures 2 and 5 are normalized to the cells prior to the initiation of the differentiation protocol (DO).

Activin A evoked an increase in the expression of CXCR4, Sox-17 and Fox-A2, in cells cultured on mouse embryonic fibroblasts and human feeder cell layers. These data suggest that human feeder cell layers are able to support the differentiation of human embryonic stem cells into cells expressing markers characteristic of the definitive endoderm lineage.

The ability of human dermal fibroblasts (D551, Figures 3 and 6), human foreskin fibroblasts (HS27, Figures 3 and 6), and the human pancreatic-derived stromal cell line disclosed in WO2006094286 (HP, Figures 3 and 6) to support the differentiation of populations of cells expressing markers characteristic of the definitive endoderm lineage, derived from populations of the human embryonic stem cell line H9 (Figure 3) and H1 (Figure 6) into cells expressing markers characteristic of the pancreatic endoderm lineage was evaluated. Populations of embryonic stem cells cultured on commercially available mouse embryonic fibroblasts (MEF-SM, Figures 3 and 6), and freshly derived mouse embryonic fibroblasts (MEF, Figures 3 and 6) were included as controls. 1µM retinoic acid, 0.25uM KAAD-Cyclopamine and FGF-10 (50ng/ml) was added to populations of cells expressing markers characteristic of the definitive endoderm lineage, derived from human embryonic stem cells cultured on the feeder cell layers. Cells were harvested after 8 days. The level of expression of markers characteristic of the pancreatic endoderm lineage were analyzed by real-time PCR (Figures 3 and 6). Results shown in Figures 3 and 6 are normalized to D0 gene expression.

Retinoic acid 0.25µM KAAD Cyclopamine, and FGF-10 treatment evoked an increase in the expression of Fox-A2, HNF-4a, HNF-6 and PDX-1, in cells cultured on mouse embryonic fibroblasts, and human feeder cell layers. These data suggest that human feeder cell layers are able to support the differentiation of cells expressing markers characteristic of the pancreatic endoderm lineage, derived from populations of the human embryonic stem cell line H9 (Figure 3) and H1 (Figure 6) into cells expressing markers characteristic of the pancreatic endoderm lineage.

The ability of human dermal fibroblasts (D551, Figures 4 and 7), human foreskin fibroblasts (HS27, Figures 4 and 7), and the human pancreatic-derived stromal cell line disclosed in WO2006094286 (HP, Figures 4 and 7) to support the differentiation of populations of cells expressing markers characteristic of the pancreatic endoderm lineage, derived from populations of the human embryonic stem cell line H9 (Figure 4) and H1 (Figure 7) into cells expressing markers characteristic of the pancreatic endocrine lineage was evaluated. Populations of embryonic stem cells cultured on commercially available mouse embryonic fibroblasts (MEF-SM, Figures 4 and 7), and freshly derived mouse embryonic fibroblasts (MEF, Figures 4 and 7) were included as controls. The γ-secretase inhibitor DAPT at 1µM, exendin-4, IGF-1 and HGF (all 50 ng/ml) were added to populations of cells expressing markers characteristic of the pancreatic endoderm lineage, derived from human embryonic stem cells cultured on the feeder cell layers. Following 9 days of culture, the level of expression of markers characteristic of the pancreatic endocrine cells were analyzed by real-time PCR (Figures 4 and 7). Results shown in Figures 4 and 7 are normalized to D0 gene expression.

γ-secretase inhibitor, exendin-4, IGF-1 and HGF treatment evoked an increase in the expression of Fox-A2, HNF-4a, HNF-6, neuro-D1, Nkx2.2, Pax-4, Nkx6.1, PDX-1, glucagon, and insulin, in cells cultured on mouse embryonic fibroblasts, and human feeder cell layers. These data suggest that human feeder cell layers are able to support the differentiation of cells expressing markers characteristic of the pancreatic endocrine lineage, derived from populations of the human embryonic stem cell line H9 (Figure 3) and H1 (Figure 6) into cells expressing markers characteristic of the pancreatic endoderm lineage. The expression of insulin and glucagon was higher in cells cultured on human feeder cell layers than on mouse feeder cell layers. These data suggest that human feeder cell layers are more able to support the differentiation of human embryonic stem cells.

## Claims

1. A method for differentiating human pluripotent stem cells, comprising the steps of:
a. Culturing the pluripotent stem cells,
b. Plating the pluripotent stem cells onto a human feeder cell layer, and
c. Treating the pluripotent stem cells on the human feeder cell layer with at least activin A followed by treatment with at least one factor that promotes the differentiation into cells expressing markers characteristic of the pancreatic endoderm lineage or of the pancreatic endocrine lineage.

2. The method of claim 1, wherein the human feeder cell layer comprises dermal fibroblast cells.

3. The method of claim 1, wherein the human feeder cell layer comprises foreskin fibroblast cells.

4. The method of claim 1, wherein the human feeder cell layer comprises pancreatic-derived stromal cells.

5. The method of claim 1, wherein the pluripotent stem cells are embryonic stem cells.

6. The method of claim 5, wherein the embryonic stem cells are human embryonic stem cells.

7. The method of claim 4, wherein the pancreatic-derived stromal cells are substantially negative in the expression of at least one protein marker selected from the group consisting of NCAM, ABCG2, cytokeratin 7, cytokeratin 8, cytokeratin 18, and cytokeratin 19.

8. The method of claim 4, wherein the pancreatic-derived stromal cells are substantially positive in the expression of at least one protein marker selected from the group consisting of CD44, CD73, CD90 and CD105.

9. The method of any one of claims 1 to 8, wherein the step of culturing the pluripotent stem cells is accomplished on an extracellular matrix.

10. The method of any one of claims 1 to 8, wherein the step of culturing the pluripotent stem cells is accomplished on a human feeder cell layer.

11. The method of any one of claims 1 to 10, wherein the at least one factor that promotes the differentiation into cells expressing markers characteristic of the pancreatic endoderm lineage or of the pancreatic endocrine lineage is selected from retinoic acid, KAAD Cyclopamine, and a fibroblast growth factor.

12. The method of any one of claims 1 to 10, wherein the at least one factor that promotes the differentiation into cells expressing markers characteristic of the pancreatic endoderm lineage or of the pancreatic endocrine lineage is selected from exendin-4, IGF-1 and HGF.

## Patentansprüche

1. Verfahren zur Differenzierung menschlicher pluripotenter Stammzellen, umfassend die Schritte:
a. Kultivieren der pluripotenten Stammzellen,
b. Ausplattieren der pluripotenten Stammzellen auf eine menschliche Feeder-Zellschicht und
c. Behandeln der pluripotenten Stammzellen auf der menschlichen Feeder-Zellschicht mit wenigstens Activin A, gefolgt von einer Behandlung mit wenigstens einem Faktor, der die Differenzierung zu Zellen, die für die pankreatische Endodermlinie oder die pankreatische Endokrinlinie charakteristische Marker exprimieren, fördert.

2. Verfahren nach Anspruch 1, wobei die menschliche Feeder-Zellschicht Hautfibroblasten-Zellen umfasst.

3. Verfahren nach Anspruch 1, wobei die menschliche Feeder-Zellschicht Vorhautfibroblasten-Zellen umfasst.

4. Verfahren nach Anspruch 1, wobei die menschliche Feeder-Zellschicht aus Pankreas stammende Stromazellen umfasst.

5. Verfahren nach Anspruch 1, wobei es sich bei den pluripotenten Stammzellen um embryonale Stammzellen handelt.

6. Verfahren nach Anspruch 5, wobei es sich bei den embryonalen Stammzellen um menschliche embryonale Stammzellen handelt.

7. Verfahren nach Anspruch 4, wobei die aus Pankreas stammenden Stromazellen weitgehend negativ bei der Expression wenigstens eines aus der Gruppe bestehend aus NCAM, ABCG2, Cytokeratin 7, Cytokeratin 8, Cytokeratin 18 und Cytokeratin 19 ausgewählten Proteinmarkers sind.

8. Verfahren nach Anspruch 4, wobei die aus Pankreas stammenden Stromazellen weitgehend positiv bei der Expression wenigstens eines aus der Gruppe bestehend aus CD44, CD73, CD90 und CD105 ausgewählten Proteinmarkers sind.

9. Verfahren nach einem der Ansprüche 1 bis 8, wobei der Schritt der Kultivierung der pluripotenten Stammzellen auf einer extrazellulären Matrix erfolgt.

10. Verfahren nach einem der Ansprüche 1 bis 8, wobei der Schritt der Kultivierung der pluripotenten Stammzellen auf einer menschlichen Feeder-Zellschicht erfolgt.

11. Verfahren nach einem der Ansprüche 1 bis 10, wobei der wenigstens eine Faktor, der die Differenzierung zu Zellen, die für die pankreatische Endodermlinie oder die pankreatische Endokrinlinie charakteristische Marker exprimieren, fördert, aus Retinsäure, KAAD-Cyclopamin und einem Fibroblasten-Wachstumsfaktor ausgewählt ist.

12. Verfahren nach einem der Ansprüche 1 bis 10, wobei der wenigstens eine Faktor, der die Differenzierung zu Zellen, die für die pankreatische Endodermlinie oder die pankreatische Endokrinlinie charakteristische Marker exprimieren, fördert, aus Exendin-4, IGF-1 und HGF ausgewählt ist.

## Revendications

1. Procédé destiné à différencier des cellules souches pluripotentes humaines, comprenant les étapes consistant à :
a. Cultiver les cellules souches pluripotentes,
b. Ensemencer sur plaque les cellules souches pluripotentes sur une couche de cellules nourricières humaines, et
c. Traiter les cellules souches pluripotentes sur la couche de cellules nourricières humaines avec au moins une activine A, suivie par un traitement avec au moins un facteur qui favorise la différenciation en cellules exprimant des marqueurs caractéristiques de la lignée de l'endoderme pancréatique ou de la lignée endocrine pancréatique.

2. Procédé de la revendication 1, dans lequel la couche de cellules nourricières humaines comprend des cellules de fibroblastes dermiques.

3. Procédé de la revendication 1, dans lequel la couche de cellules nourricières humaines comprend des cellules de fibroblastes de prépuce.

4. Procédé de la revendication 1, dans lequel la couche de cellules nourricières humaines comprend des cellules stromales dérivées du pancréas.

5. Procédé de la revendication 1, dans lequel les cellules souches pluripotentes sont des cellules souches embryonnaires.

6. Procédé de la revendication 5, dans lequel les cellules souches embryonnaires sont des cellules souches embryonnaires humaines.

7. Procédé de la revendication 4, dans lequel les cellules stromales dérivées du pancréas sont substantiellement négatives pour l'expression d'au moins un marqueur protéique choisi dans le groupe constitué par les NCAM, ABCG2, cytokératine 7, cytokératine 8, cytokératine 18 et cytokératine 19.

8. Procédé de la revendication 4, dans lequel les cellules stromales dérivées du pancréas sont substantiellement positives pour l'expression d'au moins un marqueur protéique choisi dans le groupe constitué par les CD44, CD73, CD90 et CD105.

9. Procédé de l'une quelconque des revendications 1 à 8, dans lequel l'étape de culture des cellules souches pluripotentes est accomplie sur une matrice extracellulaire.

10. Procédé de l'une quelconque des revendications 1 à 8, dans lequel l'étape de culture des cellules souches pluripotentes est accomplie sur une couche de cellules nourricières humaines.

11. Procédé de l'une quelconque des revendications 1 à 10, dans lequel l'au moins un facteur qui favorise la différenciation en cellules exprimant des marqueurs caractéristiques de la lignée de l'endoderme pancréatique ou de la lignée endocrine pancréatique est choisi parmi l'acide rétinoïque, la Cyclopamine-KAAD et un facteur de croissance des fibroblastes.

12. Procédé de l'une quelconque des revendications 1 à 10, dans lequel l'au moins un facteur qui favorise la différenciation en cellules exprimant des marqueurs caractéristiques de la lignée de l'endoderme pancréatique ou de la lignée endocrine pancréatique est choisi parmi l'exendine-4, l'IGF-1 et le HGF.
